# EUROPÄISCHE PATENTSCHRIFT

(11) **EP 1 084 097 B1**
(45) Veröffentlichungstag und Bekanntmachung des Hinweises auf die Patenterteilung: **14.08.2002**
(21) Anmeldenummer: 99926442.7
(22) Anmeldetag: 27.05.1999
(51) Int. Cl.: C07C 51/47, C07C 53/21, B01J 47/00

(54) **VERFAHREN ZUR RÜCKGEWINNUNG VON FLUORIERTEN ALKANSÄUREN AUS ABWÄSSERN**
METHOD FOR RECOVERING FLUORINATED ALKANOIC ACIDS FROM WASTE WATERS
PROCEDE POUR LA RECUPERATION D'ACIDES ALCANOIQUES FLUORES DANS DES EAUX RESIDUAIRES

(30) Priorität: 02.06.1998 DE 19824614
(43) Veröffentlichungstag der Anmeldung: 21.03.2001
(60) Teilanmeldung: 02075150.9
(73) Patentinhaber: Dyneon GmbH & Co. KG, 84504 Burgkirchen (DE); Axiva GmbH, 65926 Frankfurt am Main (DE)
(72) Erfinder: FELIX, Bernd, D-84508 Burgkirchen (DE); SULZBACH, Reinhard, D-84489 Burghausen (DE); FÜHRER, Stephan, D-84508 Burgkirchen (DE); KAISER, Thomas, D-65779 Kelkheim (DE); KNIEP, Hagen, D-65931 Frankfurt am Main (DE); BUDESHEIM, Armin, D-65207 Wiesbaden (DE)
(74) Vertreter: Voortmans, Gilbert J.L.
(86) Internationale Anmeldenummer: EP9903672
(87) Internationale Veröffentlichungsnummer: WO9962858

(56) Entgegenhaltungen:
- EP-A- 0 014 431
- US-A- 3 882 153
- US-A- 4 369 266
- US-A- 5 017 480

## Beschreibung

Für die Polymerisation fluorierter Monomerer in wäßriger Dispersion werden fluorierte Alkansäuren als Emulgatoren eingesetzt, da sie keine telogenen Eigenschaften haben. Vor allem werden die Salze, vorzugsweise die Alkali- oder Ammoniumsalze, von perfluorierten oder teilfluorierten Alkancarbonsäuren oder -sulfonsäuren verwendet. Diese Verbindungen werden durch Elektrofluorierung oder durch die Telomerisation fluorierter Monomerer hergestellt, was mit hohem Aufwand verbunden ist. Es hat deshalb nicht an Versuchen gefehlt, diese Wertstoffe aus Abwässern wiederzugewinnen.

Aus der US-A-5 442 097 ist ein Verfahren zur Rückgewinnung von fluorierten Carbonsäuren in verwertbarer Form aus verunreinigten Ausgangsmaterialien bekannt, wobei man aus diesen Materialien im wäßrigen Medium mit einer hinreichend starken Säure die fluorierte Carbonsäure nötigenfalls freisetzt, diese mit einem geeigneten Alkohol umsetzt und den gebildeten Ester abdestilliert. Als Ausgangsmaterial kann hierbei eine Polymerisationsflotte dienen, insbesondere aus der sogenannten Emulsionspolymerisation, bei der das Fluorpolymer in Form kolloidaler Teilchen mit Hilfe relativ hoher Mengen an Emulgator hergestellt wird. Dieses Verfahren hat sich sehr gut bewährt, setzt aber eine gewisse Konzentration an fluorierter Carbonsäure im Ausgangsmaterial voraus.

Aus der US-A-4 369 266 ist es bekannt, ein Permeat aus der Ultrafiltration von Fluorpolymer-Dispersionen, das fluorierte und stabilisierende Emulgatoren enthält, über basische Austauscherharze zu leiten, in denen der fluorierte Emulgator zurückgehalten und durch anschließende Elution gewonnen wird.

Aus der DE-A-20 44 986 ist ein Verfahren zur Gewinnung von Perfluorcarbonsäuren aus verdünnter Lösung bekannt, wobei man die verdünnte Lösung der Perfluorcarbonsäuren in Adsorptionskontakt mit einem schwachbasischen Anionenaustauscherharz bringt und dadurch die in der Lösung enthaltene Perfluorcarbonsäure an das Anionenaustauscherharz adsorbiert, das Anionenaustauscherharz mit einer wäßrigen Ammoniaklösung eluiert und damit die adsobierte Perfluorcarbonsäure in das Elutionsmittel überführt und schließlich die Säure aus dem Eluat gewinnt. Für eine vollständige Elution werden jedoch relativ große Mengen an verdünnter Ammoniaklösung benötigt und außerdem ist dieses Verfahren sehr zeitraubend. Diese Nachteile überwindet das aus der US-A-4 282 162 bekannte Verfahren zur Elution von an basischen Anionenaustauschern adsorbierten fluorierten Emulgatorsäuren, bei dem die Elution der adsorbierten fluorierten Emulgatorsäure aus dem Anionenaustauscher mit einem Gemisch aus verdünnter Mineralsäure und einem organischen Lösungsmittel vorgenommen wird. Bei diesem Verfahren wird durch den Einsatz der Säure gleichzeitig die Regeneration des Austauscherharzes bewirkt.

Es wurde gefunden, daß dieses letztgenannte Verfahren vor allem dann in der betrieblichen Praxis Probleme aufwirft, wenn das verarbeitete Abwasser sehr feinteilige Feststoffe enthält, die oft bisher nicht oder zumindest nicht als störend erkannt worden waren. In diesem Falle setzen sich die das Anionenaustauscherharz enthaltenden Apparaturen mehr oder weniger schnell mit diesen Feststoffen zu, was sich durch erhöhten Strömungswiderstand und verminderte Leistungsfähigkeit bemerkbar macht. Die üblichen vorgeschalteten Filter oder Fritten sind hierbei wirkungslos.

Es wurde außerdem gefunden, daß diese Schwierigkeiten dadurch bedingt sind, daß die feinteiligen Feststoffe durch die Emulgatorsäuren in einer relativ stabilen kolloidalen Feinverteilung gehalten werden. Wenn nun diese Säuren durch das Anionenaustauscherharz aus dem System entfernt werden, wird diese relativ stabile Feinverteilung gestört und der Feststoff fällt aus und verstopft das Austauscherharz. Es wurde somit weiterhin gefunden, daß die Leistungsfähigkeit des aus der US-A-4 282 162 bekannten Verfahrens erheblich gesteigert werden kann und auch für Abwässer geeignet ist, die feinteilige Feststoffe enthalten, wenn man diese Feststoffe aus dem Abwasser entfernt, bevor man dieses mit dem Anionenaustauscherharz in Kontakt bringt.

Ein weiterer Aspekt der Erfindung ist darin zu sehen, daß nicht nur bereits vorhandene Feststoffe entfernt werden können, sondern auch andere störende Bestandteile, die sich in Feststoffe überführen lassen. Solche störenden Bestandteile können andere Säuren beziehungsweise deren Salze sein, die ebenfalls an das Austauscherharz gebunden werden und somit nicht nur Austauscherkapazität binden, sondern gegebenenfalls auch besondere Vorkehrungen bei und/oder nach der Elution der Emulgatorsäuren erfordern.

Ein Beispiel für derartige störende Säure ist die Oxalsäure, die häufig als Puffer eingesetzt wird. Durch Zusatz von Calciumionen in stöchiometrischen Mengen oder im Über- beziehungsweise Unterschuß, beispielsweise als Chlorid oder Hydroxid, kann die Oxalsäure als schwerlösliches Oxalat ganz oder teilweise ausgefällt werden, vorteilhaft zusammen mit gegebenenfalls vorhandenen weiteren störenden feinverteilten Stoffen.

Die Erfindung bezieht sich somit auf ein Verfahren zur Gewinnung von fluorierten Emulgatorsäuren aus Abwasser, das dadurch gekennzeichnet ist, daß man zunächst aus dem Abwasser feinteilige Feststoffe und/oder in Feststoffe überführbare Anteile entfernt, anschließend die fluorierten Emulgatorsäuren an ein Anionenaustauscherharz bindet und aus diesem die fluorierten Emulgatorsäuren eluiert. Weitere Aspekte der Erfindung und ihre bevorzugten Ausgestaltungen werden im folgenden näher erläutert.

Als Abwässer kommen Prozeßabwässer in Betracht, in denen oberflächenaktive fluorierte Alkansäuren enthalten sind. Das Verfahren ist geeignet für Abwässer aus der Polymerisation fluorierter Monomerer nach dem sogenannten Emulsionsverfahren, bei dem das fluorierte Monomere unter mildem Rühren unter Einsatz einer relativ hohen Konzentration an fluorierter Emulgatorsäure in ein feinteiliges Polymeres überführt wird, das in feindisperser, kolloidaler Form vorliegt und wobei der so gewonnene Latex nach Erreichen der gewünschten Feststoffkonzentration, beispielsweise durch intensives Rühren, koaguliert wird, wodurch sich das Polymer als feines Pulver abscheidet.

Es wurde gefunden, daß bei der bekannten Aufarbeitung vor allem relativ niedermolekulare Polymeranteile Schwierigkeiten machen, wobei sich diese niedermolekularen Polymeren besonders störend bemerkbar machen, wenn das Polymerisationsverfahren zu einer breiten Molgewichtsverteilung führt. Auch bei solchen "schwierigen" Abwässern zeigt sich die Leistungsfähigkeit des erfindungsgemäßen Verfahrens.

Die Entfernung der feinteiligen Feststoffe richtet sich nach den jeweiligen Gegebenheiten:

Bei sauren Abwässern kann es genügen, eine - gegebenenfalls teilweise - Neutralisation mit geeigneten Basen wie Calciumhydroxid vorzunehmen, wobei das Kolloid - und eventuell vorhandene fällbare Stoffe wie Oxalationen - ausgefällt wird, die Emulgatorsäure oder ihr Salz jedoch in Lösung bleiben.

Eine andere Möglichkeit zur Fällung der störenden Kolloide besteht in der Zugabe von geeigneten Metallsalzen, beispielsweise Aluminiumsalze wie Aluminiumchlorid und Aluminiumsulfat, Calciumsalze wie Calciumchlorid, Magnesiumsalze wie Magnesiumchlorid und Magnesiumsulfat, Eisensalze wie Eisen(II)- oder Eisen(III)-chlorid und Eisensulfat. Bei sauren Abwässern ist auch die Zugabe entsprechender Metalle wie Aluminium, Eisen oder Magnesium möglich. Zur Verbesserung der Flockung können noch geringe Mengen eines Flockungshilfsmittel zugegeben werden.

Vorteilhaft wird das Abwasser zur Rückgewinnung reiner fluorierter Emulgatorsäuren mit sehr niedrigem Oxalsäuregehalt unter intensiver Vermischung mit einer Aluminiumsalzlösung behandelt, anschließend mit einer Kalkmilchlösung einen pH-Wert zwischen 6 und 7,5 einstellt, den sich bildenden Niederschlag abfiltriert und die mit Schwefelsäure auf einen pH-Wert unter 7 eingestellte Lösung über einen Ionenaustauscher führt.

Eine weitere Möglichkeit zur Fällung der störenden Kolloide besteht in der Elektrokoagulation. Hierbei wird an das Abwasser zur Koagulation der kolloidalen Teilchen ein elektrisches Feld angelegt. Die Teilchen schlagen sich im Falle inerter Elektroden (zum Beispiel Titan) auf den Flächen nieder. Bei löslichen Elektroden (zum Beispiel Eisen und/oder Aluminium) werden Metallkationen mit großem Ladungs- und Durchmesserverhältnis in die Lösung eingetragen, die die Koagulation wie bei der Zugabe von Metallsalzen bewirken. Vorteilhaft bei der Elektrokoagulation ist die Vermeidung des zusätzlichen Eintrags von Anionen wie beispielsweis Chlorid oder Sulfat. Zur Verbesserung der Flockung können noch geringe Mengen eines Flockungshilfsmittels zugegeben werden.

Geeignete mechanische Methoden zur Entfernung der feinteiligen Feststoffe bestehen in der Querstromfiltration (zum Beispiel mit Membranen, Zentrifugen), Tiefenfiltration (zum Beispiel Sandbettfilter) oder auch Anschwemmfiltration mit Zugabe eines Filterhilfsmittels (zum Beispiel Zellstoffe, Perlite, Kieselguren).

Die Abtrennung der ausgefällten Feststoffe kann in an sich bekannter Weise erfolgen, beispielsweise durch Filtration, nötigenfalls mit einem Filtrierhilfsmittel, durch Dekantieren, durch Flotation oder Sedimentation.

Die Adsorption der Emulgatorsäuren an Austauscherharze kann in an sich bekannter Weise erfolgen. Geeignet sind insbesondere stark basische Anionenaustauscherharze, wie sie beispielsweise unter den Handelsbezeichnungen ®AMBERLITE IRA-402, ®AMBERJET 4200 (beide: Rohm & Haas), ®PUROLITE A845 (Purolite GmbH) oder ®LEWATIT MP-500 (Bayer AG) erhältlich sind.

Die Adsorption kann in an sich bekannter Weise erfolgen, wobei die Austauscherharze in den üblichen Apparaturen wie Röhren oder Säulen angeordnet sind, die von dem Abwasser durchströmt werden:

Die Elution der gebundenen Emulgatorsäuren erfolgt ebenfalls in an sich bekannter Weise, wobei das Verfahren nach der US-A-4 282 162 bevorzugt wird.

Für die Gewinnung der Emulgatorsäuren in der erforderlichen hohen Reinheit für den Einsatz in der Polymerisation eignen sich beispielsweise die Verfahren nach der obengenannten US-A-5 442 097 oder das Verfahren nach der US-A-5 312 935, bei dem man das Eluat zunächst weitgehend wasserfrei macht und dann mit Oxidationsmitteln behandelt.

Das nach der Adsorption der Emulgatorsäuren verbleibende Abwasser wird je nach Gehalt an sonstigen Stoffen in bekannter Weise aufgearbeitet oder in den Prozeß zurückgeführt. Falls gewünscht, können noch restliche Anteile an fluorierten Emulgatorsäuren mit üblichen Adsorbenzien wie Aktivkohle entfernt werden.

Die Erfindung wird in den folgenden Beispielen noch näher erläutert.

### Beispiel 1

Als Ausgangsmaterial dient ein Abwasser aus der Copolymerisation von Tetrafluorethylen (TFE) und Perfluor(n-propyl-vinyl)ether (PPVE), bei der das Ammoniumsalz der n- und iso-Perfluoroctansäure (PFOS) im Molverhältnis 9 : 1 als Emulgator eingesetzt ist. Die PFOS-Konzentration in der Flotte beträgt 1200 mg/l, die Oxalsäure-Konzentration 1600 mg/l.

In einem Rührgefäß werden 14 l der Flotte mit 1,5 g/l einer 10gew.%igen Aluminiumchloridlösung versetzt und intensiv gerührt. Der sich bildende Niederschlag wird abfiltriert.

Etwa 50 ml eines handelsüblichen stark basischen Ionenaustauscherharzes (®AMBERLITE IRA-402, Rohm & Haas; Styrol-Divinylbenzol-Typ, Anion: Chlorid, Gel, Totalkapazität 1,3 eq/l, Schüttgewicht 710 g/l) werden in eine mit Glasfritte versehene zylindrische Glassäule (Länge 25 cm, Durchmesser 16 mm) gegeben und mit Wasser gespült. Zur Beladung des Ionentauschers wird die vorbehandelte Flotte mit einer Pumpe im Aufstrom und mit einer Lineargeschwindigkeit von 1 m/h durch die Säule gefördert, das austretende Wasser gesammelt und die PFOS-Konzentration zur Bilanzierung bestimmt. Nach der Beladung wird die Säule mit 100 ml Wasser gespült.

Zur Regeneration des Ionentauschers werden 150 ml eines Gemisches aus 89 Gew.-% Methanol, 7 Gew.-% konzentrierter Schwefelsäure und 4 Gew.-% Wasser mit einer Lineargeschwindigkeit von 0,5 m/h durch die Säule gefördert und das Eluat aufgefangen. Anschließend wird die Säule mit 100 ml Wasser gespült.

Das Eluat enthält 85 % der im Abwasser enthaltenen Emulgatorsäure und Oxalsäure mit einer Konzentration von 3900 mg/l.

### Beispiel 2

In einem Rührgefäß werden 14 l der Flotte wie in Beispiel 1 mit 1,5 g/l einer 10gew.%igen Aluminiumchloridlösung versetzt und intensiv gerührt. Anschließend wird mit einer 10gew.%igen Kalkmilchlösung ein pH-Wert von 7,5 eingestellt. Der sich bildende Niederschlag wird abfiltriert und der pH-Wert der Lösung mit verdünnter Schwefelsäure auf pH 4 eingestellt.

Aufbau und Versuchsablauf von Beladung und Regneration des Ionentauschers erfolgen analog Beispiel 1.

Das Eluat enthält hierbei 95 % der im Abwasser enthaltenen Emulgatorsäure und Oxalsäure mit einer Konzentration von 1 mg/l.

### Beispiel 3

In einem Rührgefäß werden 16 l eines Abwassers aus der Polymeraufarbeitung fluorhaltiger Polymerer vorgelegt. Bei der Polymerisation wird das Ammoniumsalz der PFOS als Emulgator eingesetzt, die PFOS-Konzentration beträgt 1200 mg/l. Zu dieser Lösung werden 2 g einer 10gew.%igen Aluminiumchloridlösung zugegeben und intensiv gerührt. Anschließend wird mit einer 10gew.%igen Kalkmilchlösung ein pH-Wert von 7,5 eingestellt und 3 mg/l eines Flockungshilfsmittels (®PRAESTOL A 3015 L, Stockhausen GmbH & Co. KG; Polyacrylamid) zugegeben. Der sich bildende Niederschlag wird abfiltriert und der pH-Wert mit Schwefelsäure auf pH 4 eingestellt.

Die Beladung und Regneration des Ionentauschers erfolgen wie in Beispiel 1.

Das Eluat enthält hierbei 91 % der im Abwasser enthaltenen Emulgatorsäure.

### Vergleichsbeispiel

Als Ausgangsmaterial wird eine Mutterflotte aus der Copolymerisation von TFE und PPVE, bei der das Ammoniumsalz der PFOS als Emulgator eingesetzt wird, verwendet. Die PFOS-Konzentration beträgt 1200 mg/l.

Etwa 50 ml des im Beispiel 1 genannten stark basischen Ionenaustauscherharzes werden in eine mit Glasfritte versehene zylindrische Glassäule (Länge 25 cm, Durchmesser 16 mm) gegeben und mit Wasser gespült. Zur Beladung des Ionentauschers wird die Flotte unbehandelt mit einer Pumpe im Aufstrom durch das Bett gefördert. Der Druckverlust über das Ionentauscherbett wird mit einem Manometer gemessen. Der Beladungsversuch mußte nach Überleiten von 400 ml der Flotte abgebrochen werden, da infolge von ausgefallenem Polymer das Harz verklebte.

## Patentansprüche

1. Verfahren zur Gewinnung von fluorierten Emulgatorsäuren aus Abwasser, **dadurch gekennzeichnet, daß** man zunächst aus dem Abwasser aus der Polymerisation fluorierter Monomerer feinteilige Feststoffe und/oder in Feststoffe überführbare Anteile entfernt, anschließend die fluorierten Emulgatorsäuren an ein Anionenaustauscherharz bindet und aus diesem die fluorierten Emulgatorsäuren eluiert.

2. Verfahren nach Anspruch 1, **dadurch gekennzeichnet, daß** die feinteiligen Feststoffe ausgefällt werden.

3. Verfahren nach Anspruch 1, **dadurch gekennzeichnet, daß** die feinteiligen Feststoffe mechanisch entfernt werden.

4. Verfahren nach Anspruch 1, **dadurch gekennzeichnet, daß** die in Feststoffe überführbaren Anteile ausgefällt werden.

5. Verfahren nach einem oder mehreren der Ansprüche 1, 2 und 4, **dadurch gekennzeichnet, daß** die gefällten Stoffe durch Sedimentation abgetrennt werden.

6. Verfahren nach einem oder mehreren der Ansprüche 1, 2 und 4, **dadurch gekennzeichnet, daß** die gefällten Stoffe durch Flotation abgetrennt werden.

7. Verfahren nach einem oder mehreren der vorhergehenden Ansprüche, **dadurch gekennzeichnet, daß** man zur Rückgewinnung reiner fluorierter Emulgatorsäuren mit sehr niedrigem Oxalsäuregehalt das Abwasser unter intensiver Vermischung mit einer Aluminiumsalzlösung behandelt, anschließend mit einer Kalkmilchlösung einen pH-Wert zwischen 6 und 7,5 einsteilt, den sich bildenden Niederschlag abfiltriert und die mit Schwefelsäure auf einen pH-Wert unter 7 eingestellte Lösung über einen Ionenaustauscher führt.

8. Verfahren nach einem oder mehreren der vorhergehenden Ansprüche, **dadurch gekennzeichnet, daß** das eingesetzte Anionenaustauscherharz stark basisch ist.

9. Verfahren nach einem oder mehreren der vorhergehenden Ansprüche, **dadurch gekennzeichnet, daß** die Elution der fluorierten Emulgatorsäure aus dem Anionentauscher mit einem Gemisch aus verdünnter Mineralsäure und einem organischen Lösungsmittel erfolgt.

## Claims

1. Process for the recovery of fluorinated emulsifier acids from wastewater, **characterized in that** fine solids and/or material which can be converted into fine solids are removed from the wastewater from the polymerization of fluorinated monomers, the fluorinated emulsifier acids are subsequently bound on an anion-exchange resin and the fluorinated emulsifier acids are eluted from the latter.

2. Process according to Claim 1, **characterized in that** the fine solids are precipitated.

3. Process according to Claim 1, **characterized in that** the fine solids are mechanically removed.

4. Process according to Claim 1, **characterized in that** the material which is convertible into solids is precipitated.

5. Process according to one or more of Claims 1, 2 and 4, **characterized in that** the precipitated materials are separated off by sedimentation.

6. Process according to one or more of Claims 1, 2 and 4, **characterized in that** the precipitated materials are separated off by flotation.

7. Process according to one or more of the preceding claims, **characterized in that** for the recovery of pure fluorinated emulsifier acids having a very low oxalic acid content, the wastewater is treated with an aluminium salt solution while mixing intensively, the pH is subsequently adjusted to a value in the range from 6 to 7.5 by means of milk of lime, the precipitate which forms is filtered off and, after the pH of the solution is adjusted to a value below 7 using sulphuric acid, the solution is passed over an ion exchanger.

8. Process according to one or more of the preceding claims, **characterized in that** the anion-exchange resin used is a strong base anion-exchange resin.

9. Process according to one or more of the preceding claims, **characterized in that** elution of the fluorinated emulsifier acid from the anion-exchange resin is carried out using a mixture of dilute mineral acid and an organic solvent.

## Revendications

1. Procédé d'obtention d'acides émulsifiants fluorés à partir d'eaux résiduaires, **caractérisé en ce que** l'on élimine tout d'abord, de l'eau résiduaire, les substances solides finement divisées provenant de la polymérisation de monomères fluorés et/ou les portions convertissables en substances solides, **en ce que** l'on fixe ensuite les acides émulsifiants fluorés sur une résine échangeuse d'anions et **en ce que** l'on procède à l'élution hors de cette dernière des acides émulsifiants fluorés.

2. Procédé selon la revendication 1, **caractérisé en ce que** les substances solides finement divisées sont précipitées.

3. Procédé selon la revendication 1, **caractérisé en ce que** les substances solides finement divisées sont éliminées par voie mécanique.

4. Procédé selon la revendication 1, **caractérisé en ce que** les portions convertissables en substances solides sont précipitées.

5. Procédé selon l'une quelconque ou plusieurs des revendications 1, 2, et 4, **caractérisé en ce que** les substances précipitées sont séparées par sédimentation.

6. Procédé selon l'une quelconque ou plusieurs des revendications 1, 2 et 4, **caractérisé en ce que** les substances précitées sont séparées par flottation.

7. Procédé selon l'une quelconque ou plusieurs des revendications précédentes, **caractérisé en ce qu'**en vue de la récupération des acides émulsifiants fluorés purs à teneur en acide oxalique très faible, on traite l'eau résiduaire sous mélange intensif à l'aide d'une solution de sel d'aluminium, **en ce que** l'on ajuste ensuite une valeur de pH comprise entre 6 et 7,5 à l'aide d'une solution de lait de chaux, **en ce que** l'on sépare par filtration le précipité qui se forme et **en ce que** l'on fait passer la solution ajustée à une valeur de pH inférieure à 7 à l'aide d'acide sulfurique sur un échangeur d'ions.

8. Procédé selon l'une quelconque ou plusieurs des revendications précédentes, **caractérisé en ce que** la résine échangeuse d'anions utilisée est très fortement basique.

9. Procédé selon l'une quelconque ou plusieurs des revendications précédentes, **caractérisé en ce que** l'élution des acides émulsifiants fluorés hors de l'échangeu d'anions se fait à l'aide d'un mélange d'un acide minéral dilué et d'un solvant organique.
